# EUROPEAN PATENT APPLICATION

(11) **EP 3 594 332 A1**
(43) Date of publication of application: **15.01.2020**
(21) Application number: 18382514.0
(22) Date of filing: 10.07.2018
(51) Int. Cl.: C12N 9/08, C07K 14/375, C12N 15/52, C12N 15/63, C12N 15/70, C12N 15/72, C12N 15/74

(54) **METHOD OF HETEROLOGOUS EXPRESSION OF ACTIVE FUNGAL UNSPECIFIC PEROXYGENASE IN BACTERIAL HOST CELLS FOR FATTY-ACID EPOXIDATION AND OTHER OXYGENATION REACTIONS**

(71) Applicant: Consejo Superior de Investigaciones Cientificas, 28006 Madrid (ES)
(72) Inventor: FERNÁNDEZ FUEYO, Elena, 28040 MADRID (ES); ARANDA OLIDEN, Carmen, 41012 SEVILLA (ES); GUTIÉRREZ SUÁREZ, Ana, 41012 SEVILLA (ES); MARTÍNEZ FERRER, Angel T., 28040 MADRID (ES)
(74) Representative: Pons Ariño, Angel

(57) **Abstract**

The present invention relates to a method for heterologous expression of active fungal unspecific peroxygenase and/or variants thereof in bacterial host cells, preferably in *Escherichia coli.* The invention also relates to the recombinant active fungal unspecific peroxygenase and/or variants thereof thus obtained and to the use thereof. The invention further relates to a method for epoxidation of unsaturated fatty acids, or oxyfunctionalization of other organic compounds, comprising the recombinant active fungal unspecific peroxygenase and/or variants thereof obtainable by the method disclosed herein.

## Description

The present invention relates to a method for heterologous expression of active fungal unspecific peroxygenase and/or variants thereof in bacterial host cells, preferably in *Escherichia coli.* The invention also relates to the recombinant active fungal unspecific peroxygenase and/or variants thereof thus obtained and to the use thereof. The invention further relates to a method for epoxidation of unsaturated fatty acids, or oxyfunctionalization of other organic compounds, comprising the recombinant active fungal unspecific peroxygenase and/or variants thereof obtainable by the method disclosed herein.

### BACKGROUND ART

Among industrial oxidoreductases (Martinez A.T., et al. Biotechnol. Adv. 2017; 35, 815-831), fungal unspecific peroxygenases (UPOs; EC 1.11.2.1) are recently-described enzymes with high interest as industrial biocatalysts (Hofrichter M., et al. Adv. Exp. Med. Biol. 2015; 851, 341-368). The first UPO was described in 2004 in the basidiomycete *Agrocybe aegerita* (AaeUPO) as a haloperoxidase. Further characterization showed its ability to incorporate oxygen into aromatic substrates. Later, its oxygenation activity on aliphatic substrates was reported (Gutiérrez A., et al., Arch. Biochem. Biophys. 2011; 514, 33-43), and it was classified as unspecific peroxygenase (EC 1.11.2.1).

Unfortunately, only three additional UPOs have been purified from cultures of the basidiomycetes *Marasmius rotula* (*Mro*UPO) (Gröbe G., et al., AMB Express. 2011; 1, 31-42.) and *Coprinellus radians* (*Cra*UPO) (Anh D.H., et al., Appl. Environ. Microbiol. 2007; 73, 5477-5485.) and the ascomycete *Chaetomium globosum* (*Cgl*UPO) (Kiebist J.,et al., Chem. Bio. Chem., 2017; 18, 563-569). One more UPO from the genome of the basidiomycete *Coprinopsis cinerea* has been expressed in *Aspergillus* by Novozymes (r*Cci*UPO) as an industrial biocatalyst (Babot E.D., et al., Biotechnol. Bioeng. 2013; 110, 2332). On the other hand, more than 2000 putative UPO genes have been identified in fungal genomes, being impossible to characterize them due to the lack of a heterologous expression system.

One of the most challenging reaction in organic synthesis is the selective introduction of oxygen functionalities (in C-H bonds). The resulting products, regio- and enantio-selectively oxygenated organic molecules, have a great importance for the fine chemical and pharmaceutical industry. Chemical oxygenation methods have a slight control on the selectivity of the reaction, which is driven by the intrinsic properties of the substrate. However, some enzymes catalyze highly regio/stereo selective oxyfunctionalization reactions, with enantiomeric excesses higher than 99%. One class of these extraordinary selective enzymes are UPOs (Wang, Y., et al. Curr. Opin. Chem. Biol. 2017;37: 1-9.) being *Mro*UPO one of the members with the highest biotechnological interest (Aranda, C., et al. Catal. Sci. Technol. 2018; 8, 2394-2401; Olmedo, A., et al. Chem.-Eur.J. 2017; 23, 16985-16989).

Until now, only two UPOs, *Aae*UPO and *Cci*UPO, have been expressed heterologously as active enzymes, always in eukaryotic hosts, and one of them (*Aae*UPO) only as a mutated variant after directed evolution for increasing the expression yield. WO2017081355A1 specifically claims the production of an evolved *Aae*UPO (including some mutations required for heterologous expression) in yeast hosts (of the genera *Saccharomyces and Pichia*); and WO2015079064A2 the production of CciUPO and engineered variants in an *Aspergillus* hosts.

On the other hand, several fungal peroxidases, related to the peroxygenases considered in the present patent, have been expressed in *Escherichia coli as* protein aggregates, called inclusion bodies, which have to be solubilized and laboriously refolded to obtain active enzyme (Perez-Boada, M., et al. Enzyme Microb. Technol. 2002; 30: 518-524; Miki, Y., et al. Protein Express. Purif. 2009; 68: 208-214; and Linde, D., et al. Protein Express. Purif. 2014; 103: 28-37). Unfortunately, refolding yields are very low, making the recombinant production of these enzymes in *Escherichia coli* not suitable for commercial purposes. Incredibly, more than ten years after UPO discovery, there are no methods for the production of recombinant peroxygenases in prokaryotic systems as soluble active enzymes or by *in vitro* activation. Therefore, in the state of the art there is a need for an easy, fast and robust bacterial expression system for the production of recombinant peroxygenases, especially in active form.

### SUMMARY OF THE INVENTION

The present invention relates to a method of expression of recombinant UPO, and/or variants or mutants thereof, in a prokaryotic system as a soluble active enzyme, preferably in a bacterial host cell, and more preferably in *Escherichia coli.*

*Escherichia coli* is the best expression system for the rational design of enzymes in general, allowing a rapid collection, purification and evaluation of recombinant enzymes and variants or mutants thereof.

For the purposes of the present invention, the term "peroxygenase" relates to the unspecific peroxygenase (UPO) enzyme in accordance with EC 1.11.2.1, which catalyses the insertion of an oxygen atom from H₂O₂ or other organic hydroperoxides, which acts as a source of oxygen and final electron acceptor, in a wide variety of substrates. For purposes of the present invention, peroxygenase activity is determined using veratryl alcohol (VA), 2,2'-azino-bis(3-ethylbenzothiazoline-6-sulphonic acid (ABTS) and 2,6-dimethoxyphenol (DMP) as substrates, according to Ullrich, R., et al. (Appl. Environ. Microbiol. 2004; 70, 4575-4581).

For the purposes of the present invention, the term "mutant" or "variant", used indistinctly throughout the text, relates to the UPO enzymes comprising an alteration, i.e., a substitution, insertion, and/or deletion, at one or several positions, which can be obtained by means of the methods described herein. A substitution means replacement of the amino acid occupying a position with a different amino acid; a deletion means removal of the amino acid occupying a position; and an insertion means adding an amino acid at a certain position. The variants of the present invention often have improved activity on a substrate of interest. For purposes of the present invention, any mutant or variant known in the art can be used in the method of the present invention. Non-limiting examples of UPO mutants or variants are disclosed in Gómez de Santos et al. (ACS Catal. 2018; 8, 4789-4799), Molina-Espeja et al. (ChemBioChem 2016; 17, 341-349) and WO2017081355A1.

The term "wild-type" peroxygenase means a peroxygenase as expressed by a naturally occurring microorganism, such as a filamentous fungus found in nature.

Thus, in a first aspect, the present invention relates to a method for expressing an active fungal UPO or variants thereof as a soluble active enzyme, into a bacterial host cell, as shown in **Example 1,** wherein the method comprising the following steps:
a) introducing an expression vector encoding the mature unspecific peroxygenase and a signal peptide or a fragment thereof, or variants thereof, into the bacterial host cell, preferably into *Escherichia coli,*
b) cultivating the bacterial host cell of step (a) under conditions conducive for production of the polypeptide as an active enzyme, wherein the culture medium comprises an inducer and a compound containing heme, and the temperature for the culture is selected from a range of 10 to 25 °C; and
c) recovering and purifying the recombinant enzyme.

The terms "coding" or "encoding" sequence means a polynucleotide, which directly specifies the amino acid sequence of the enzyme or variant. The boundaries of the coding sequence are generally determined by an open reading frame, which begins with a start codon such as ATG, GTG or TTG and ends with a stop codon such as TAA, TAG, or TGA. The coding sequence may be a genomic DNA, cDNA, synthetic DNA, or a combination thereof.

Thus, the term "encoding a fungal UPO" refers herein to a nucleotide sequence encoding a mature fungal UPO and can further comprise nucleotides encoding for a signal peptide or a fragment thereof. The nucleotide sequence encoding for a mature fungal UPO can be selected from any nucleotide sequence encoding for a fungal UPO, or any variants thereof known in the art, that can be optimized for Escherichia coli codon usage (e.g. using the OPTIMIZER web server, Puigbo, P., et al. Nucleic Acids Res. 2007; 35:W126-W131).

In a preferred embodiment the mature unspecific peroxygenase is selected from the list consisting of: *Marasmius rotula* comprising the SEQ ID NO: 1, *Chaetomium globosum* comprising the SEQ ID NO: 2, *Agrocybe aegerita* comprising the SEQ ID NO: 3, *Coprinopsis cinerea* comprising the SEQ ID NO: 4, and/or any variants thereof. In a more preferred embodiment, the mature unspecific peroxygenase comprises the SEQ ID NO: 1 (UPO from *Marasmius rotula*).

The term "signal peptide", as used in the description, relates to a peptide which is located at the amino end of a polypeptide or protein, and whose function is to direct the localisation of the protein at different compartments of the cell (nucleus, mitochondria, chloroplast, endoplasmic reticulum, Golgi apparatus, etc.) or to the extracellular space, in the case that the protein is secreted.

In a preferred embodiment the signal peptide of the present invention can be selected from the native signal peptide from the specific fungal UPO or any variants thereof known in the art and also mentioned above. In a more preferred embodiment, the signal peptide is selected from the list consisting of: amino acids -1 to -28 from SEQ ID NO: 1 (UPO from *Marasmius rotula),* -1 to -18 from SEQ ID NO: 2 (UPO from *Chaetomium globosum),* -1 to -46 from SEQ ID NO: 3 (UPO from *Agrocybe aegerita),* -1 to -50 from SEQ ID NO: 4 (UPO from *Coprinopsis cinerea),* and/or any combinations thereof. In a more preferred embodiment, the signal peptide is the amino acids -1 to -28 from SEQ ID NO: 1 (UPO from *Marasmius rotula*)*.* The preferred signal peptide of the present invention (comprising the amino acids -1 to -28 from SEQ ID NO: 1) favours the functional expression of the active UPO obtainable by the method of the invention.

The nucleotide sequence encoding the recombinant active fungal UPO obtained by the method of the present invention may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression. The expression vectors referred to in the present invention comprise a polynucleotide encoding the fungal UPO described herein, a promoter, and transcriptional and translational stop signals. The various nucleic acids and control sequences described herein may be joined together to produce a recombinant expression vector which may include one or more convenient restriction sites to allow for insertion or substitution of the nucleotide sequence encoding the enzyme at such sites.

The terms "vector" or "expression vector" relate to the vehicle (e.g., a plasmid or virus) whereby a DNA or RNA sequence (for example, a heterologous gene) can be introduced in a host cell, for the purpose of transforming the host and promoting the expression (for example, transcription and translation) of the sequence introduced. The vectors typically comprise the DNA of a transmissible agent, wherein the foreign DNA encodes a protein inserted using restriction enzyme technology. A common type of vector is a "plasmid", which is generally a double-stranded DNA molecule, which can easily accept additional DNA (foreign) and that can be easily introduced in a suitable host cell.

A large number of vectors have been described for replication and/or expression in a variety of hosts. Non-limiting examples include pKK plasmids (Clonetech), pUC plasmids, pRSET or PrEP plasmids (Invitrogen, San Diego, CA), pMAL plasmids (New England Biolabs, Beverly, MA), pGAPZaA, pcWori+, pET-26b (+), pXTD14, pYEX-S1, pMAL and pET-22b (+), or the pET-15b and pET-23a plasmids (Novagen, Inc., Madison, WI) used in the present invention. In a preferred embodiment, the expression vector is the pET-23a plasmid. Recombinant vectors often include one or more replication systems for cloning or expression, one or more markers for selection in the host, for example, resistance to antibiotics, and one or more expression cassettes. Suitable vectors for insertion of said polynucleotide are vectors derived from expression vectors in prokaryotes such as, by way of example, pET, pUC18, pUC19, Bluescript and its derivatives, mp18, mp19, pBR322, pMB9, Co1E1, pCR1, RP4, phages and "launch" vectors, such as pSA3 and pAT28. Other vectors can be used as desired by a person skilled in the art. Routine experimentation in biotechnology can be used to determine the most suitable vectors for use with the invention, if different to that described in the **Examples.** In general, the choice of the vector depends on the size of the polynucleotide and of the host cell to be used in the methods of this invention.

The term "control sequences" is defined herein to include all the necessary components for the expression of the polypeptide coding sequences of the present invention. Each control sequence may be native or foreign to the nucleotide sequence that encodes the native or foreign polypeptide there between. Such control sequences include, but are not limited to, a leader, polyadenylation sequence, pro-peptide sequence, promoter, signal peptide sequence and transcription terminator. The control sequences include, at least, a promoter and translation and transcription stop signals. The control sequences may have links in order to introduce specific restriction sites that facilitate the linkage of the control sequences with the coding region of the nucleotide sequence that encodes a polypeptide.

A "promoter sequence" is a DNA regulatory region capable of binding to the RNA polymerase in a cell and initiating the transcription of a gene (direction 3') downstream from the coding sequence. For the purpose of defining this invention, the promoter sequence is limited at its 3' terminus by the transcription start site and extends upstream (5' direction) to include the minimum number of necessary bases or elements to begin the transcription at detectable levels above the base.

The expression "operationally linked" relates to a juxtaposition wherein the components thus described have a relationship that allows them to function intentionally. A control sequence "operationally linked" to a coding sequence is linked in such a manner that the expression of the coding sequence is achieved under conditions compatible with the control sequences.

More than one copy of a polynucleotide encoding the recombinant active fungal UPO obtained by the method of the present invention may be inserted into the host cell to increase the production of the gene product. An increase in the copy number of the polynucleotide can be obtained by integrating at least one additional copy of the sequence into the host cell genome or by including an amplifiable selectable marker gene with the polynucleotide, where cells containing amplified copies of the selectable marker gene, and thereby additional copies of the polynucleotide, can be selected by cultivating the cells in the presence of the appropriate selectable agent. The procedures used to link the elements described above to construct the recombinant expression vectors referred to in the present invention are well known to one skilled in the art.

As used in the present specification, a "host cell" includes any culturable cell that can be modified through the introduction of DNA not contained naturally in the cell. Preferably, a host cell is that in which the nucleotide discloses in the invention can be expressed, giving rise to a stable polypeptide located in the appropriate subcellular compartment. The choice of an appropriate host cell can also be influenced by the choice of the detection signal. For example, the use of constructions with reporter genes (for example, lacZ, luciferase, thymidine kinase or GFP) can provide a selectable signal by activating or inhibiting the transcription of the gene of interest in response to a transcription-regulating protein. The phenotype of the host cell must be considered in order to achieve an optimal selection or screening.

The host cell of the present invention is preferably a bacterial host cell. The bacterial host cell may be any bacterial cell useful in the recombinant production of the active fungal UPO or variants thereof, including Gram-positive or Gram-negative bacteria. Gram- positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, Escherichia, Flavobacterium, Fusobacterium, Helicobacter, Ilyobacter, Neisseria, Pseudomonas and Salmonella.* The bacterial host cell may be preferably *Escherichia coli.*

In a second step (b), the method of the present invention comprising the culture of the bacterial host cell of step (a) under conditions conducive for production of the recombinant UPO or variant thereof. In a more preferred embodiment, the cultured conditions for the highly production of the recombinant UPO or variant thereof comprising the use of an auto-induction system, preferably a lactose auto-induction system, further supplemented with a compound containing heme, at low temperature, preferably in a range from 10 to 25°C.

In a more preferred embodiment of the method of the present invention, the lactose auto-induction media is preferably ZYM-5052, whose composition comprises 1 % N-Z-amine AS, 0.5 % yeast extract, 25 mM Na₂HPO₄, 25 mM KH₂PO₄, 50 mM NH₄ Cl, 5 mM Na₂SO₄, 2 mM MgSO₄, 0.5 % glycerol, 0.05 % glucose, 0.2 % α-lactose. The final concentration of glucose/lactose/glycerol can be modified to optimize the expression. Besides, other complex media with adequate proportion of glucose, lactose and glycerol can be employed.

In a more preferred embodiment, the use of a lactose auto-induction medium results in a slow release of the inducing molecule (allolactose).

In a more preferred embodiment of the method of the present invention, the compound containing heme is selected from the list including, but not limited to: heme, hemin, hematin, hemochrome, hemoglobin and blood powder. In a more preferred embodiment, the compound containing heme is hemin.

In a more preferred embodiment, the compound containing heme, preferably hemin, is used in a concentration in the range from 20 to 1000 µM, preferably 100-200 µM, more preferably 200 µM, in the culture medium. The use of the compound containing heme, preferably hemin, for the production of recombinant active fungal unspecific peroxygenase according to the present invention, facilitates the incorporation of the heme group that acts as an enzyme cofactor.

In a more preferred embodiment, the low temperature of the culture ranges from 16°C to 20°C, preferably, 16°C. The expression temperature of the method of the present invention allows keeping the soluble protein in active form instead of accumulating in inclusion bodies.

In a third step (c), the method of the present invention comprises recovering and purifying the recombinant polypeptide.

Identification of the recombinant active fungal UPO obtained by the method of the present invention has been confirmed by methods known in the art that are specific for enzymes and other polypeptides. These detection methods may include formation of an enzyme product or disappearance of an enzyme substrate. Preferably, the identification of the recombinant active fungal UPO obtained by the method of the present invention may be performed by electrophoresis, N-terminal sequencing and crystallization.

The resulting recombinant active fungal UPO may be recovered using methods known in the art including, but not limited to, centrifugation, filtration, extraction, spray-drying, evaporation, or precipitation.

The recombinant active fungal UPO produced by the method of the present invention may be purified by a variety of procedures known in the art including, but not limited to, chromatography (e.g., ion exchange, cation exchange, affinity, hydrophobic, chromatofocusing, high performance liquid chromatography -HPLC- and size exclusion), electrophoretic procedures (e.g., preparative isoelectric focusing, and polyacrylamide gel electrophoresis, PAGE), differential solubility (e.g., ammonium sulfate precipitation), or extraction, in order to obtain a substantially pure and active UPO.

The use of a nucleotide sequence that, in addition to the mature protein, also encodes all or part of the signal peptide from the UPO protein, and a lactose auto-induction medium supplemented with hemin and cultured at low temperature, preferably 16°C, allows a good expression of recombinant fungal UPOs or variants thereof into bacterial host cell, preferably into *Escherichia coli,* as active enzymes.

The recombinant active UPOs obtained by the method of the present invention have the same properties as the non-recombinant, hereinafter wild-type, enzymes in terms of activity, stability and regio- and enantio-selectivity. Therefore, the feasibility of the method has been shown in the present invention. Consequently, the production of recombinant UPOs and/or variants thereof in a bacterial system allows the easy generation of mutants and the up-scaled production of the enzymes or variants thereof obtained by the method disclosed in the present invention.

The present invention also shows the expression of active UPO variants or mutants by the method disclosed herein. In this sense, the variants mentioned herein are contributing to understand important aspects of catalysis by the model UPO from *Marasmius rotula* - such as the importance of dimerization at Cys227, and the involvement of Glu157 in the enzyme activation by peroxide, as shown in **Example 3.**

The advantage of the present invention is: i) the shorter production times (3 days) compared to the wild-type enzymes naturally produced by the fungi and the recombinant enzymes produced in eukaryotic systems (7-15 days) resulting in a reduction in cost and energy consumption; ii) the possibility to express the true wild-type enzymes, when required, compared to the evolved (mutated) variants expressed in yeast; and iii) the possibility to express engineered variants, when required, compared to the wild-type enzymes naturally produced by the fungi.

The method of active expression of the present invention is also much less costly than other common bacterial expression systems, used for related heme-enzymes in which the protein accumulates as inclusion bodies and *in vitro* activation is required.

The method of the present invention can be applied to the production of recombinant active fungal UPOs such as wild-type enzyme, evolved variants, mutants, etc, from a fast growing bacterial host cell, such as *Escherichia coli.*

Therefore, the present invention provides new peroxygenases showing all the aforementioned advantages over native or wild-type peroxygenase, related to the active heterologous expression in a bacterial host cell, preferably *Escherichia coli.*

Thus, in a second aspect, the present invention also refers to the recombinant active fungal UPO or variants thereof obtainable by the method of the present invention.

Peroxygenases are known for their large number of applications in organic synthesis, where they have been described as the "dream enzymes" (Wang, Y, Curr. Opin. Chem. Biol. 2017; 37, 1-9) for a variety of selective hydroxylation and other oxyfuctionalization reactions in fine chemistry, production of drugs and drug metabolites, and even in some bulk chemistry reactions (Hofrichter et al., Adv. Exp. Med. Biol. 2015; 851, 341-368; and Martinez, A.T., Biotechnol. Adv. 2017; 35, 815-831). Thus, the recombinant active fungal obtainable by the method of the present invention may have any of the currently known uses for these enzymes in the state of the art.

Therefore, another aspect of the present invention refers to a method for the oxyfunctionalization of organic compounds comprising contacting an organic compound with a peroxide and the recombinant unspecific peroxygenase, or variants hereof, obtainable by the method disclosed in the present invention.

In a more preferred embodiment of the method for the oxyfunctionalization, the organic compounds are selected from the list consisting of, but not limited to: fatty acids, fatty aldehydes, fatty alcohols, alkanes, alkenes, alkynes, organic sulfides, monoaromatics, polyaromatics and heterocycles. In the same sense, the organic compound can be linear or cyclic, saturated or unsaturated and carry different functional groups.

In a preferred embodiment, the oxyfunctionalization reaction is an enzymatic reaction and is selected from the list consisting of: hydroxylation, epoxidation or sulfoxidation.

In a preferred embodiment, the peroxide has a hydrogen atom as one of its substituents -hydroperoxide-, and is selected from, but not limited to, the list consisting of hydrogen peroxide, *tert*-butyl hydroperoxide, triphenyl silyl hydroperoxide, cumyl hydroperoxide, or any combination of these hydroperoxides. Preferably the hydro peroxide is hydrogen peroxide.

Moreover, as described below, the oxygenation reactions catalyzed by the recombinants UPO obtained by the method of the present invention, preferably the *Mro*UPO (r*Mro*UPO), comprises highly efficient epoxidation of unsaturated fatty acids, a reaction rarely catalyzed by other industrial enzymes that has not been reported before for any other wild or recombinant fungal UPO.

Oils and fats are among the most important renewable feedstock of the chemical industry. Among them, unsaturated fatty acids and their methyl esters can epoxidized, a reaction of great industrial interest (Corma, A., et al. Chem. Rev. 2007; 107, 2411-2502). The industrial-scale epoxidation of unsaturated fatty-acid compounds is generally carried out by the Prileshajev reaction via percarboxylic acids. However, this method, which often includes strong mineral acids as catalysts for the *in situ* generation of peracids, suffers from several drawbacks such as the relatively low selectivity for epoxides due to oxirane ring opening in the acidic medium, the corrosive nature of acids, and the unstable character of peracids (Danov, S.M., et al. Catal. Sci. Technol. 2017; 7, 3659-3675).

Many studies have been aimed at searching an alternative, such as the chemo-enzymatic synthesis with lipases catalyzing the carboxylic acid reaction with hydrogen peroxide (Björkling, F., et al. Tetrahedron 1992; 48, 4587-4592). However, the latter reaction maintains most drawbacks of peracid-based epoxidation. Therefore, direct enzymatic processes emerge as an alternative solution for more selective and environmentally friendly epoxidation of unsaturated lipids. Several enzymes are known to catalyze epoxidation directly, such as cytochrome P450 monooxygenases. However, they present some drawbacks, such as their intracellular nature, and the requirement for costly co-substrates in the two former cases. As illustrated in **Example 2,** recombinant UPOs obtained by the method disclosed herein represent a promising enzymatic technology to epoxidize unsaturated fatty acids under mild and environmentally-friendly conditions, as potential alternative to the above chemical and enzymatic epoxidations. These peroxygenases elude some of the limitations of other monooxygenases since they are far more stable and only require H₂O₂ for activation.

In summary, the method of the present invention can be further applied to the production of recombinant active UPOs, such as MroUPO (SEQ ID NO: 1) *Cgl*UPO (SEQ ID NO: 2), AaeUPO (SEQ ID NO: 3) and *Cci*UPO (SEQ ID NO: 4), - switching from the current wild enzymes (the only one available for *Cgl*UPO), evolved variants from yeast hosts, known in the art and also available together with wild enzyme for AaeUPO, and *Aspergillus*-expressed enzymes (*Cci*UPO) to recombinant *wild-type* (i.e. non-mutated) or engineered UPOs from a fast growing bacterium, preferably *Escherichia coli.* The invention also describes the use of the recombinant peroxygenases obtained by the method of the present invention as a biocatalyst in the production of reactive epoxides from unsaturated fatty acids, and other oxygenation reactions of interest for the green chemistry and bio-based industrial sectors.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting the present invention.

### DESCRIPTION OF THE DRAWINGS

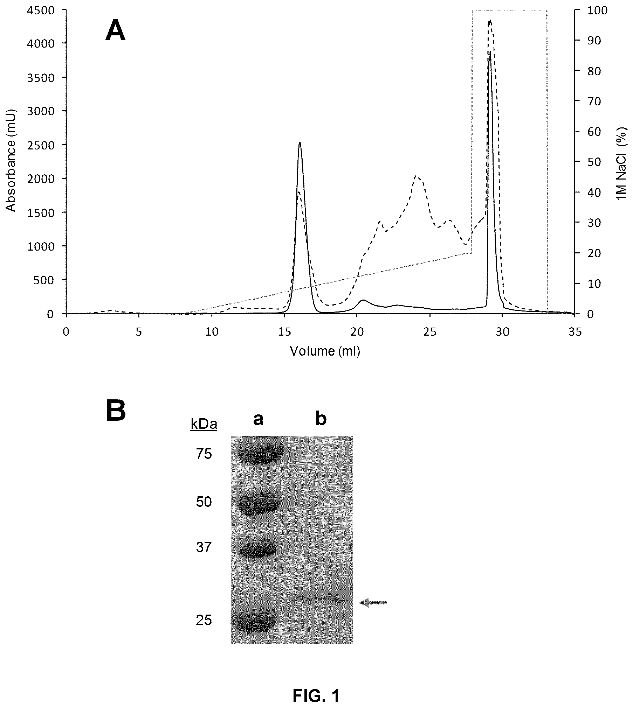

**FIG. 1****.** Purification of r*Mro*UPO heterologously expressed in *Escherichia coli as* a soluble active enzyme. (**A**) Mono S chromatography showing 280 nm (dashed line) and 410 nm (continuous line) absorbance profiles and NaCl concentration (dotted line). (**B**) SDS-PAGE of the purified enzyme r*Mro*UPO (lane b, arrow) compared with different molecular mass standards (lane a).

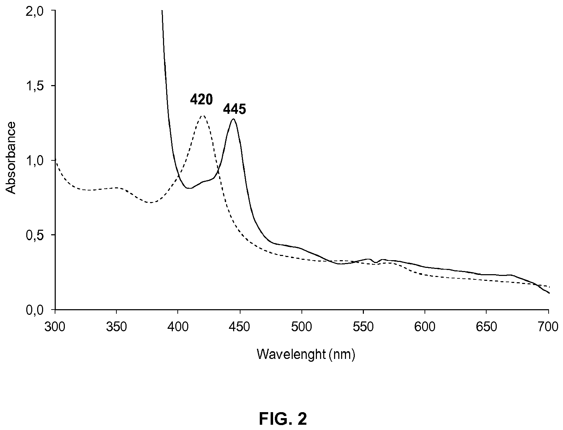

**FIG. 2****.** UV-visible spectra of r*Mro*UPO (dashed line) and its complex with carbon monooxide (continuous line). Main (Soret) maxima are indicated.

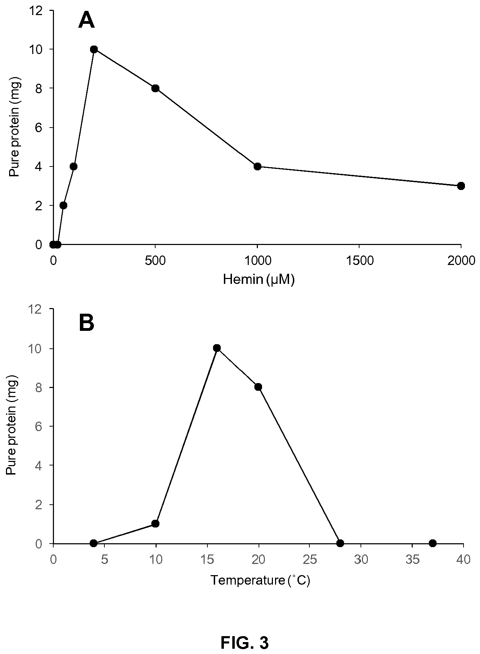

**FIG. 3****.** Effect of hemin concentration (**A**) and temperature (**B**) in the production of active r*Mro*UPO after 60 h in ZYM-5052 lactose auto-inducible medium, at 20°C (**A**) and 200 µM hemin concentration (**B**).

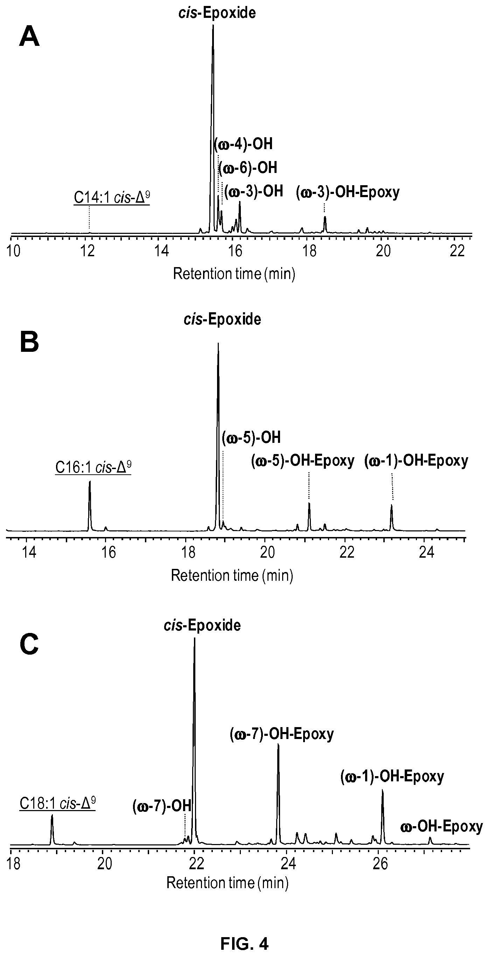

**FIG. 4****.** Gas chromatography-mass spectrometry (GC-MS) analysis of r*Mro*UPO reactions with unsaturated myristoleic (C14:1 cis-Δ⁹; **A**), palmitoleic (C16:1 cis-Δ⁹; **B**) and oleic (C18:1 cis-Δ⁹; **C**) fatty acids, showing the remaining substrate (underlined) and the different epoxy (*cis*-epoxide), hydroxy-epoxy (at ω, ω-1, ω-3, ω-5 and ω-7 positions), and hydroxyl derivatives (from ω-3 to ω-7 positions).

### Examples

Following are examples of the invention by means of assays carried out by the inventors, which evidence the effectiveness of the product of the invention. These examples serve to illustrate the invention and must not be considered to limit the scope thereof.

### Example 1. Production of Marasmius rotula UPO in Escherichia coli as an active enzyme.

In order to be able to express the *Mro*UPO coding sequence in *Escherichia coli* as an active enzyme, two different pET vectors, pET-23a and pET-15b (the latter introducing His-tags), and two different nucleotide sequence (SEQ ID NO: 5) encoding *Mro*UPO (SEQ ID NO: 1) protein with and without signal peptide, respectively (signal peptide from *Mro*UPO is the residues -1 to -28 of the SEQ ID NO: 1) were used, which were optimized for *Escherichia coli* codon usage using the OPTIMIZER web server (Puigbo, P., et al. Nucleic Acids Res. 2007; 35: W126-W131). Additionally, different concentrations of hemin in the culture media and different growth temperatures were also tested for the expression of r*Mro*UPO in *Escherichia coli.*

The resulting plasmids pET-23a-*Mro*UPO and pET-15b-*Mro*UPO, each of them with and without the signal peptide encoding nucleotide sequence, were used for the expression in BL21(DE3)pLysS *Escherichia coli.* Cells were grown for 60 hours in ZYM-5052 lactose auto-inducible medium comprising 1 % N-Z-amine AS, 0.5 % yeast extract, 25 mM Na₂HPO₄, 25 mM KH₂PO₄, 50 mM NH₄Cl, 5 mM Na₂SO₄, 2 mM MgSO₄, 0.5 % glycerol, 0.05 % glucose and 0.2 % α-lactose, supplemented with a range of 0-2000 µM hemin concentrations, at a range of temperatures from 4°C to 37°C. Cells were harvested by centrifugation at 10000 rpm for 10 minutes at 4°C. Bacteria were resuspended in 10 mM phosphate pH 7, supplemented with lysozyme (0.5 mg/mL) and DNase I. After 30 minutes of incubation, cells were sonicated, and debris was removed by centrifugation at 20,000 rpm for 4 h. The supernatant was concentrated with Amicon of 50-kDa cut-off, and dialyzed against 10 mM phosphate, pH 7.

Recombinant *Mro*UPO (r*Mro*UPO) was purified using an AKTA high-performance liquid chromatography (HPLC) system, in three consecutive steps. The first separation was performed on a 6 mL Resource™ Q cartridge using 10 mM phosphate (pH 7). Peroxygenase activity was followed by 2,6-dimethoxyphenol (DMP) oxidation in the presence of H₂O₂. The appropriate fractions were pooled, concentrated and dialyzed against 10 mM phosphate (pH 7), and loaded into a Mono Q high-resolution 5/5 column at a flow rate of 1 mL/min. Finally, the appropriate fractions were pooled, concentrated and dialyzed against 10 mM citrate (pH 4) and loaded into a Mono S high-resolution 5/5 column (**FIG. 1A**).

r*Mro*UPO purification was confirmed by sodium dodecyl sulfate-polyacrylamide gel electrophoresis (SDS-PAGE) in 12 % gels stained with Coomassie Brilliant Blue R-250, showing a single band with a molecular mass of -28 kDa (**FIG. 1B**). The electronic absorption spectra of the purified enzyme and its complex with carbon monooxide were recorded with an Agilent 8453 diode array UV-visible spectrophotometer, showing the typical displacement of the Soret peak (**FIG. 2**). With the above approach, 10 mg/L of pure active r*Mro*UPO with the same catalytic and selectivity properties of wild-type UPO (naturally produced by the fungus) can be produced in *Escherichia coli* shaken flasks. The use of fermenters increases the protein production.

The functional expression of r*Mro*UPO described above, was obtained with the pET-23a expression vector and the DNA sequence of *Mro*UPO including signal peptide (whole SEQ ID NO: 1). Despite many attempts using (i) pET-15b and *Mro*UPO with and without signal peptide DNA sequence, and (ii) pET-23a and *Mro*UPO DNA sequence without signal peptide sequence, no protein expression was achieved under these conditions. These results indicate a role of the signal peptide for the expression of active *Mro*UPO.

Additionally, the role of the hemin in the culture media for the expression of r*Mro*UPO was also analysed. UPOs as well as P450 monoxygenases contain heme as prosthetic group, hemin addition being essential for the correct folding and activity of the protein. In order to enhance the production of the r*Mro*UPO, different concentrations of hemin were added to the culture media. As it is shown in **FIG. 3A****,** the highest protein production was obtained using 200 µM of hemin, and no protein was produced in the absence of external supply of hemin.

Furthermore, the effect of the temperature in the culture media for the expression of r*Mro*UPO was also analysed. Different temperatures were screened for the optimization of the expression of active r*Mro*UPO. As it is shown in **FIG. 3B****,** the maximum protein production was achieved at the lowest temperature, 16 °C.

Moreover, a similar efficiency was obtained at 20 °C. On the other hand, the typical temperatures used for growing *Escherichia coli,* such as 37 °C and 28 °C, completely impaired the expression of active r*Mro*UPO.

As in the case of the auto-induction expression system, low temperatures contribute to reduce the metabolism of *Escherichia coli,* improving the expression of active r*Mro*UPO avoiding its accumulation in inclusion bodies. Finally, up to 10 mg of pure r*Mro*UPO in active form can be obtained by liter of recombinant *Escherichia coli* culture, as described above. For comparative purposes, the amount of parental (wild-type) AaeUPO expressed in recombinant *Saccharomyces cerevisiae* cultures is of only 0.007 mg/L, which could be increased up to 8 mg/L by protein directed evolution (Molina-Espeja, P., et al. Appl.Environ.Microbiol. 2014; 80, 3496-3507).

### Example 2. Epoxidation of unsaturated fatty acids by rMroUPO obtained by the method of the invention.

Three unsaturated fatty acids - myristoleic (C14:1 cis-Δ⁹), palmitoleic (C16:1 cis- Δ⁹) and oleic (C18:1 cis-Δ⁹) acids - were treated with rMrUPO obtained by the method of the present invention (**Example 1**), and the products analyzed by GC-MS. The enzymatic reactions were performed at 0.1 mM substrate concentration (1 mL reaction volume) and 30°C, in 50 mM phosphate buffer (pH 5.5). Prior to use, the substrate was dissolved in acetone and added to the buffer to give an acetone concentration in the reaction of 20% (v/v). Enzyme and H₂O₂ concentration were 0.17 µM and 2.5 mM respectively. In control experiments, substrates were treated under the same conditions (including H₂O₂) but without enzyme.

After 30 min reaction, products of unsaturated fatty acid conversion were extracted with methyl *tert*-butyl ether and dried under N₂. N,O-Bis(trimethylsilyl)trifluoroacetamide (BSTFA; Supelco) was used to prepare trimethylsilyl (TMS) derivatives of unsaturated fatty acid reaction products, which were analyzed by GC-MS.

The GC-MS analyses were performed with a Shimadzu GC-MS QP 2010 Ultra system, using a fused-silica DB-5HT capillary column (30 m x 0.25 mm internal diameter, 0.1 µm film thickness) from J&W Scientific. The oven was heated from 120°C (1 min) to 300°C at 5°C·min⁻¹ and held for 15 min. The injection was performed at 300°C and the transfer line was kept at 300°C. Compounds were identifying by mass fragmentography and comparing their mass spectra with those of the Wiley and NIST libraries and standards.

As shown in **FIG. 4****,** the conversion rates were high and fatty acid epoxides were the main products in all the reactions, together with minor amounts of hydroxylated fatty acid derivatives at the ω-3, ω-4, ω-5, ω-6 and ω-7 positions. In the three conversions, the cis-epoxide was the main product obtained. However, different amounts of hydroxylated epoxides - ω, ω-1, ω-3, ω-5 and ω-7 hydroxyepoxides - were also obtained, being more abundant in the oleic acid reaction. The r*Mro*UPO obtained by the method of the present invention also epoxidizes the above unsaturated fatty acids as methyl and glyceryl esters, as well as the polyunsaturated (e.g. linoleic) fatty acids.

### Example 3. Generation and use of rMroUPO variants.

The main advantage and the most remarkable application of the expression method of the present invention is the possibility of easily producing mutants or variants with enhanced properties regarding wild-type UPO enzymes. These enhance properties can be in terms of stability, activity, regio/stero-selectivity and substrate scope. Moreover, mutagenesis studies combined with computational simulations and the availability of the crystal structure of the *Mro*UPo heterologously produced in *Escherichia coli* will improve the understanding of this new class of enzymes.

Using the heterologous functional expression system disclosed in the present invention three mutated variants of *Mro*UPO have been already obtained (**Table 1**). These mutations were introduced in the *Mro*UPO sequence by polymerase chain reaction (PCR) using the expression plasmid pET-23a-*Mro*UPO (see below) as template, and the QuikChange kit from Stratagene (La Jolla, CA, USA). The 5'-GGC GAT AAC *GC*C GGC GCT ATC GTT CTC AGC CCG G-3' (C227A) (SEQ ID NO: 6), 5'- C CGC TCT TCA GAA TCT GCG *GC*C TTC CTG ACC G -3' (F160Y) (SEQ ID NO: 7), and 5'-C CGC TCT TCA G*C*A TCT GCG TTC TTC CTG ACC G-3'(E157A) (SEQ ID NO: 8) direct primers (mutated codons, underlined, and nucleotides, italics, are indicated), and the reverse primers bearing the complementary sequences were synthesized.

The PCR reactions (50 µl volume) were carried out in an Eppendorf (Hamburg, Germany) Mastercycler pro-S thermal cycler using 20 ng of template DNA, 500 µM each dNTP, 125 ng direct and reverse primers, 2.5 units of *Pfu*Turbo polymerase (Stratagene), and the manufacturer's buffer. Reaction conditions included: **i)** a start cycle of 1 min at 95°C; **ii)** 18 cycles of 50 s at 95°C, 50 s at 55°C, and 10 min at 68°C; and **iii)** a final cycle of 10 min at 68°C. The mutated genes were expressed in *Escherichia coli* and the variants purified as described above for the native (recombinant non-mutated) *Mro*UPO (**Example 1**).

**Table 1. Mutated variants of MroUPO produced**

| **Mutation** | **Protein (mg L⁻¹)** |
|---|---|
| C227A | 4 |
| F160Y | 14.5 |
| E157A | 7.5 |

With these mutations, we have been able to understand some crucial aspects of oxygenation catalysis by r*Mro*UPO:
- r*Mro*UPO is only active as a dimer: Mutation C227A, removing intermolecular disulfide bond, results in inactive enzyme,
- E157 is implicated in the reaction with H₂O₂ being essential for the activity of the enzyme, as shown by activity loss after the E157A mutation,
- F160Y varies the selectivity of *Mro*UPO stopping oxygenation of:
   i. fatty acids at the alcohol (including ω-OH fatty-acid formation) or aldehyde levels, while the native enzyme overoxidizes them generating dicarboxylic acids (chromatograms available); and
   ii. stilbene yielding 4-hydroxy-stilbene, while the native enzyme immediately generates 4,4'-dihydroxy-stilbene (GC-MS analysis not shown); among other organic substrates.

## Claims

1. A method for expressing an active fungal unspecific peroxygenase, or variants thereof, into a bacterial host cell, wherein the method comprises:
a) introducing an expression vector encoding the mature unspecific peroxygenase and a signal peptide or any fragment thereof, or variants thereof, into the bacterial host cell,
b) cultivating the bacterial host cell of step (a) under conditions conducive to production of the polypeptide as an active enzyme, wherein the culture medium is a lactose auto-inducible medium further comprising a compound containing heme, and the temperature for the culture is selected from a range of 10 to 25°C, and
c) recovering and purifying the recombinant polypeptide.

2. The method according to claim 1, wherein the bacterial host cell is *Escherichia coli.*

3. The method according to any one of claims 1 to 2 wherein the expression vector is pET-23, preferably pET23a.

4. The method according to any one of claims 1 to 3 wherein the lactose auto-inducible medium is ZYM-5052.

5. The method according to any one of claims 1 to 4 wherein the compound containing heme is selected from the list consisting of: heme, hemin, hematin, hemochrome, hemoglobin and blood powder.

6. The method according to claim 5 wherein the compound containing heme is in a range of concentration between 20 to 1000 µM, preferably 100 to 200 µM.

7. The method according to any one of claims 1 to 6 wherein the temperature for the culture is selected from a range of 16 to 20°C.

8. The method according to any one of claims 1 to 7 wherein the mature unspecific peroxygenase is selected from the list consisting of: *Marasmius rotula* (SEQ ID NO: 1), *Chaetomium globosum* (SEQ ID NO: 2), *Agrocybe aegerita* (SEQ ID NO: *3), Coprinopsis cinerea* (SEQ ID NO: 4), and/or any variants thereof.

9. The method according to any of claims 1 to 8 wherein the signal peptide is selected from the list consisting of: amino acids -1 to -28 from SEQ ID NO: 1; -1 to -18 from SEQ ID NO: 2; -1 to -46 from SEQ ID NO: 3; and -1 to -50 from SEQ ID NO: 4.

10. The method according to any of claims 1 to 9 wherein the mature unspecific peroxygenase is *Marasmius rotula* (SEQ ID NO: 1) and the signal peptide is the amino acids -1 to -28 from SEQ ID NO: 1.

11. The method according to any one of claims 1 to 10 wherein the extraction and purification of the recombinant protein from the supernatant is performed by procedures selected from the list consisting of: chromatography, electrophoretic techniques and fractionated precipitation.

12. A recombinant active fungal unspecific peroxygenase or variants thereof obtainable by the method according to any one of claims 1 to 11.

13. A method for the oxyfunctionalization of organic compounds comprising contacting an organic compound with an organic hydroperoxide and the recombinant unspecific peroxygenase, or variants hereof, obtainable by the method according to any of claims 1 to 11.

14. The method for oxyfunctionalization according to claim 13 wherein the oxyfunctionalization reaction is selected from the list consisting of: hydroxylation, epoxidation and sulfoxidation.

15. The method of oxyfunctionalization according to any of claims 13 to 14 wherein the oxyfunctionalization reaction is an epoxidation and wherein the organic compounds are unsaturated fatty acids.


SEQUENCE LISTING

<110>  Consejo Superior de Investigaciones Científicas (CSIC)

<120>  METHOD OF HETEROLOGOUS EXPRESSION OF ACTIVE FUNGAL UNSPECIFIC
       PEROXYGENASE IN BACTERIAL HOST CELLS FOR FATTY-ACID EPOXIDATION
       AND OTHER OXYGENATION REACTIONS

<130>  1641.1384

<160>  8

<170>  PatentIn version 3.5

<210>  1
<211>  264
<212>  PRT
<213>  Marasmius rotula

<220>
<221>  SIGNAL
<222>  (1)..(28)

<220>
<221>  mat_peptide
<222>  (29)..(264)

<400>  1

Met Lys Leu Ala Ile Ser Ser Ser Leu Ile Ala Leu Val Ser Val Thr
            -25                 -20                 -15

Thr Ala Leu Ala Asn Ser Gln Asp Val Val Asp Phe Ser Ala His Pro
        -10                 -5                  -1  1

Trp Lys Ala Pro Gly Pro Asn Asp Ser Arg Gly Pro Cys Pro Gly Leu
5                   10                  15                  20

Asn Thr Leu Ala Asn His Gly Phe Leu Pro Arg Asn Gly Arg Asn Ile
                25                  30                  35

Ser Val Pro Met Ile Val Lys Ala Gly Phe Glu Gly Tyr Asn Val Gln
            40                  45                  50

Ser Asp Ile Leu Ile Leu Ala Gly Lys Ile Gly Met Leu Thr Ser Arg
        55                  60                  65

Glu Ala Asp Thr Ile Ser Leu Glu Asp Leu Lys Leu His Gly Thr Ile
    70                  75                  80

Glu His Asp Ala Ser Leu Ser Arg Glu Asp Val Ala Ile Gly Asp Asn
85                  90                  95                  100

Leu His Phe Asn Glu Ala Ile Phe Thr Thr Leu Ala Asn Ser Asn Pro

105                 110                 115

Gly Ala Asp Val Tyr Asn Ile Ser Ser Ala Ala Gln Val Gln His Asp
            120                 125                 130

Arg Leu Ala Asp Ser Leu Ala Arg Asn Pro Asn Val Thr Asn Thr Asp
        135                 140                 145

Leu Thr Ala Thr Ile Arg Ser Ser Glu Ser Ala Phe Phe Leu Thr Val
    150                 155                 160

Met Ser Ala Gly Asp Pro Leu Arg Gly Glu Ala Pro Lys Lys Phe Val
165                 170                 175                 180

Asn Val Phe Phe Arg Glu Glu Arg Met Pro Ile Lys Glu Gly Trp Lys
                185                 190                 195

Arg Ser Thr Thr Pro Ile Thr Ile Pro Leu Leu Gly Pro Ile Ile Glu
            200                 205                 210

Arg Ile Thr Glu Leu Ser Asp Trp Lys Pro Thr Gly Asp Asn Cys Gly
        215                 220                 225

Ala Ile Val Leu Ser Pro Glu Leu
    230                 235

<210>  2
<211>  259
<212>  PRT
<213>  Chaetomium globosum

<220>
<221>  SIGNAL
<222>  (1)..(18)

<220>
<221>  mat_peptide
<222>  (19)..(259)

<400>  2

Met Arg Thr Ser Leu Leu Pro Ala Leu Ala Ala Val Ser Pro Val Leu
            -15                 -10                 -5

Ala Gly Phe Asp Thr Trp Ala Pro Pro Gly Pro Tyr Asp Val Arg Gly
    -1  1               5                   10

Pro Cys Pro Met Leu Asn Thr Leu Thr Asn His Gly Phe Phe Pro His
15                  20                  25                  30

Asp Gly Gln Asp Ile Asp Arg Glu Thr Thr Glu Asn Ala Leu Phe Asp
                35                  40                  45

Ala Leu His Val Asn Lys Thr Leu Ala Ser Phe Leu Phe Asp Phe Ala
            50                  55                  60

Leu Thr Thr Asn Pro Ile Ala Asn Ser Thr Thr Phe Ser Leu Asn Asp
        65                  70                  75

Leu Gly Asn His Asn Val Leu Glu His Asp Ala Ser Leu Ser Arg Ala
    80                  85                  90

Asp Ala Tyr His Gly Ser Val Leu Ala Phe Asn His Thr Ile Phe Glu
95                  100                 105                 110

Glu Thr Lys Ser Tyr Trp Thr Asp Glu Thr Val Thr Leu Lys Met Ala
                115                 120                 125

Ala Asp Ala Arg Tyr Tyr Arg Ile Lys Ser Ser Gln Ala Thr Asn Pro
            130                 135                 140

Thr Tyr Gln Met Ser Glu Leu Gly Asp Ala Phe Thr Tyr Gly Glu Ser
        145                 150                 155

Ala Ala Tyr Val Val Leu Phe Gly Asp Lys Glu Ser Gln Thr Val Pro
    160                 165                 170

Arg Ser Trp Val Glu Trp Leu Phe Glu Lys Glu Gln Leu Pro Gln His
175                 180                 185                 190

Leu Gly Trp Lys Arg Pro Ala Thr Ser Phe Glu Leu Asn Asp Leu Asp
                195                 200                 205

Lys Phe Met Ala Leu Ile Gln Asn Tyr Thr Gln Glu Ile Glu Glu Pro
            210                 215                 220

Ser Cys Glu Ser Arg Lys Gln Arg Arg Lys Pro Arg Gly Pro Ser His
        225                 230                 235

Phe Gly Phe
    240

<210>  3
<211>  371
<212>  PRT
<213>  Agrocybe aegerita

<220>

<221>  SIGNAL
<222>  (1)..(46)

<220>
<221>  mat_peptide
<222>  (47)..(371)

<400>  3

Met Lys Tyr Phe Pro Leu Phe Pro Thr Leu Val Phe Ala Ala Arg Val
    -45                 -40                 -35

Val Ala Phe Pro Ala Tyr Ala Ser Leu Ala Gly Leu Ser Gln Gln Glu
-30                 -25                 -20                 -15

Leu Asp Ala Ile Ile Pro Thr Leu Glu Ala Arg Glu Pro Gly Leu Pro
                -10                 -5                  -1  1

Pro Gly Pro Leu Glu Asn Ser Ser Ala Lys Leu Val Asn Asp Glu Ala
        5                   10                  15

His Pro Trp Lys Pro Leu Arg Pro Gly Asp Ile Arg Gly Pro Cys Pro
    20                  25                  30

Gly Leu Asn Thr Leu Ala Ser His Gly Tyr Leu Pro Arg Asn Gly Val
35                  40                  45                  50

Ala Thr Pro Val Gln Ile Ile Asn Ala Val Gln Glu Gly Leu Asn Phe
                55                  60                  65

Asp Asn Gln Ala Ala Val Phe Ala Thr Tyr Ala Ala His Leu Val Asp
            70                  75                  80

Gly Asn Leu Ile Thr Asp Leu Leu Ser Ile Gly Arg Lys Thr Arg Leu
        85                  90                  95

Thr Gly Pro Asp Pro Pro Pro Pro Ala Ser Val Gly Gly Leu Asn Glu
    100                 105                 110

His Gly Thr Phe Glu Gly Asp Ala Ser Met Thr Arg Gly Asp Ala Phe
115                 120                 125                 130

Phe Gly Asn Asn His Asp Phe Asn Glu Thr Leu Phe Glu Gln Leu Val
                135                 140                 145

Asp Tyr Ser Asn Arg Phe Gly Gly Gly Lys Tyr Asn Leu Thr Val Ala
            150                 155                 160

Gly Glu Leu Arg Phe Lys Arg Ile Gln Asp Ser Ile Ala Thr Asn Pro
        165                 170                 175

Asn Phe Ser Phe Val Asp Phe Arg Phe Phe Thr Ala Tyr Gly Glu Thr
    180                 185                 190

Thr Phe Pro Ala Asn Leu Phe Val Asp Gly Arg Arg Asp Asp Gly Gln
195                 200                 205                 210

Leu Asp Met Asp Ala Ala Arg Ser Phe Phe Gln Phe Ser Arg Met Pro
                215                 220                 225

Asp Asp Phe Phe Arg Ala Pro Ser Pro Arg Ser Gly Thr Gly Val Glu
            230                 235                 240

Val Val Ile Gln Ala His Pro Met Gln Pro Gly Arg Asn Val Gly Lys
        245                 250                 255

Ile Asn Ser Tyr Thr Val Asp Pro Thr Ser Ser Asp Phe Ser Thr Pro
    260                 265                 270

Cys Leu Met Tyr Glu Lys Phe Val Asn Ile Thr Val Lys Ser Leu Tyr
275                 280                 285                 290

Pro Asn Pro Thr Val Gln Leu Arg Lys Ala Leu Asn Thr Asn Leu Asp
                295                 300                 305

Phe Phe Phe Gln Gly Val Ala Ala Gly Cys Thr Gln Val Phe Pro Tyr
            310                 315                 320

Gly Arg Asp
        325

<210>  4
<211>  387
<212>  PRT
<213>  Coprinopsis cinerea

<220>
<221>  SIGNAL
<222>  (1)..(50)

<220>
<221>  mat_peptide
<222>  (51)..(387)

<400>  4

Met Ile Ser Thr Ser Lys His Leu Phe Val Leu Leu Pro Leu Phe Leu
-50                 -45                 -40                 -35

Val Ser His Leu Ser Leu Val Leu Gly Phe Pro Ala Tyr Ala Ser Leu

-30 -25 -20

Gly Gly Leu Thr Glu Arg Gln Val Glu Glu Tyr Thr Ser Lys Leu Pro
-15 -10 -5

Ile Val Phe Pro Pro Pro Pro Pro Glu Pro Ile Lys Asp Pro Trp Leu
-1 1 5 10

Lys Leu Val Asn Asp Arg Ala His Pro Trp Arg Pro Leu Arg Arg Gly
15 20 25 30

Asp Val Arg Gly Pro Cys Pro Gly Leu Asn Thr Leu Ala Ser His Gly
35 40 45

Tyr Leu Pro Arg Asp Gly Val Ala Thr Pro Ala Gln Ile Ile Thr Ala
50 55 60

Val Gln Glu Gly Phe Asn Met Glu Tyr Gly Ile Ala Thr Phe Val Thr
65 70 75

Tyr Ala Ala His Leu Val Asp Gly Asn Pro Leu Thr Asn Leu Ile Ser
80 85 90

Ile Gly Gly Lys Thr Arg Lys Thr Gly Pro Asp Pro Pro Pro Pro Ala
95 100 105 110

Ile Val Gly Gly Leu Asn Thr His Ala Val Phe Glu Gly Asp Ala Ser
115 120 125

Met Thr Arg Gly Asp Phe His Leu Gly Asp Asn Phe Asn Phe Asn Gln
130 135 140

Thr Leu Trp Glu Gln Phe Lys Asp Tyr Ser Asn Arg Tyr Gly Gly Gly
145 150 155

Arg Tyr Asn Leu Thr Ala Ala Ala Glu Leu Arg Trp Ala Arg Ile Gln
160 165 170

Gln Ser Met Ala Thr Asn Gly Gln Phe Asp Phe Thr Ser Pro Arg Tyr
175 180 185 190

Phe Thr Ala Tyr Ala Glu Ser Val Phe Pro Ile Asn Phe Phe Thr Asp
195 200 205

Gly Arg Leu Phe Thr Ser Asn Thr Thr Ala Pro Gly Pro Asp Met Asp
210 215 220

Ser Ala Leu Ser Phe Phe Arg Asp His Arg Tyr Pro Lys Asp Phe His
        225                 230                 235

Arg Ala Pro Val Pro Ser Gly Ala Arg Gly Leu Asp Val Val Ala Ala
    240                 245                 250

Ala Tyr Pro Ile Gln Pro Gly Tyr Asn Ala Asp Gly Lys Val Asn Asn
255                 260                 265                 270

Tyr Val Leu Asp Pro Thr Ser Ala Asp Phe Thr Lys Phe Cys Leu Leu
                275                 280                 285

Tyr Glu Asn Phe Val Leu Lys Thr Val Lys Gly Leu Tyr Pro Asn Pro
            290                 295                 300

Lys Gly Phe Leu Arg Lys Ala Leu Glu Thr Asn Leu Glu Tyr Phe Tyr
        305                 310                 315

Gln Ser Phe Pro Gly Ser Gly Gly Cys Pro Gln Val Phe Pro Trp Gly
    320                 325                 330

Lys Ser Asp
335

<210>  5
<211>  795
<212>  DNA
<213>  Marasmius rotula

<220>
<221>  sig_peptide
<222>  (1)..(84)

<400>  5
atgaaactgg cgatctcttc cagcctgatc gccctcgtct cggttaccac agctctggct      60

aacagccaag acgtagttga cttctccgca catccgtgga aagctccggg tccgaacgac     120

tctcgcggtc cgtgcccagg cctgaacact ctggctaacc acggcttcct gccgcgtaat     180

ggtcgtaaca tctccgtacc gatgattgtg aaagccggct tcgagggtta caacgtccag     240

agcgacatcc tgatcctcgc cggtaaaatc ggtatgctga cgtcgcgtga agctgatacc     300

atatcgttgg aagatctgaa actgcacggt accatcgaac acgatgcatc tctgagccgc     360

gaggatgtag cgattggtga taacctgcac ttcaacgagg caattttcac caccctggct     420

aactccaacc ctggtgcaga cgtttataac atttctagcg cggctcaggt gcagcatgat     480

cgtctggcag actctctggc gcgcaacccg aacgttacta acaccgattt aactgcgact     540

atccgctctt cagaatctgc gttcttcctg accgttatgt ccgcaggcga tccgctgcgt     600

ggtgaagcgc cgaagaaatt cgtgaacgtc ttctttcgtg aagaacgcat gccgattaaa 660

gaaggttgga aacgttccac caccccgatc actattccgc ttctgggtcc gatcattgag 720

cgcatcacgg aattatcgga ctggaaacca actggcgata actgcggcgc tatcgttctc 780

agcccggaac tgtaa 795

<210> 6
<211> 34
<212> DNA
<213> Artificial Sequence

<220>
<223> Direct primer of C227A variant

<400> 6
ggcgataacg ccggcgctat cgttctcagc ccgg 34

<210> 7
<211> 32
<212> DNA
<213> Artificial Sequence

<220>
<223> Direct primer of F160Y variant

<400> 7
ccgctcttca gaatctgcgg ccttcctgac cg 32

<210> 8
<211> 32
<212> DNA
<213> Artificial Sequence

<220>
<223> Direct primer of E157A variant

<400> 8
ccgctcttca gcatctgcgt tcttcctgac cg 32